# EUROPEAN PATENT SPECIFICATION

(11) **EP 3 287 789 B1**
(45) Date of publication and mention of the grant of the patent: **19.05.2021**
(21) Application number: 16206600.5
(22) Date of filing: 23.12.2016
(51) Int. Cl.: G01N 33/68

(54) **COMPOSITION AND KIT FOR DIAGNOSING BEHAVIORAL ADDICTION, AND METHOD OF DETECTING NEUROPILIN-2 FOR DIAGNOSIS OF BEHAVIORAL ADDICTION**
ZUSAMMENSETZUNG UND KIT ZUR DIAGNOSE VON VERHALTENSSUCHT UND VERFAHREN ZUR ERKENNUNG VON NEUROPILIN-2 ZUR DIAGNOSE VON VERHALTENSSUCHT
COMPOSITION ET TROUSSE PERMETTANT DE DIAGNOSTIQUER L'ACCOUTUMANCE COMPORTEMENTALE ET PROCÉDÉ DE DÉTECTION DE NEUROPILINE-2 POUR LE DIAGNOSTIC DE L'ACCOUTUMANCE COMPORTEMENTALE

(30) Priority: 25.08.2016 KR 20160108221
(43) Date of publication of application: 28.02.2018
(73) Proprietor: Korea Institute of Science and Technology, Seoul 02792 (KR)
(72) Inventor: LEE, Ji Eun, 02792 Seoul (KR); PARK, Yaeeun, 02792 Seoul (KR); CHEON, Dong Huey, 02792 Seoul (KR); KIM, Kyeong Yeon, 02792 Seoul (KR); HAN, Ki-Cheol, 02792 Seoul (KR); LEE, Cheolju, 02792 Seoul (KR); KIM, Dai-Jin, 06600 Seoul (KR); CHUN, Jiwon, 03631 Seoul (KR); KIM, Hye-Jung, 28165 Chungcheongbuk-do (KR); CHOI, Mi Ran, 13571 Gyeonggi-do (KR)
(74) Representative: Gill, Siân Victoria

(56) References cited:
- ROSSIGNOL M ET AL: "Genomic Organization of Human Neuropilin-1 and Neuropilin-2 Genes: Identification and Distribution of Splice Variants and Soluble Isoforms", GENO, ACADEMIC PRESS, SAN DIEGO, US, vol. 70, no. 2, 1 December 2000 (2000-12-01), pages 211-222, XP004437754, ISSN: 0888-7543, DOI: 10.1006/GENO.2000.6381
- G. P. DEMYANENKO ET AL: "NrCAM Deletion Causes Topographic Mistargeting of Thalamocortical Axons to the Visual Cortex and Disrupts Visual Acuity", JOURNAL OF NEUROSCIENCE, vol. 31, no. 4, 26 January 2011 (2011-01-26), pages 1545-1558, XP055391966, US ISSN: 0270-6474, DOI: 10.1523/JNEUROSCI.4467-10.2011
- UHL GEORGE R ET AL: "Curious cases: Altered dose-response relationships in addiction gene", PHARMACOLOGY AND THERAPEUTICS, ELSEVIER, GB, vol. 141, no. 3, 1 November 2013 (2013-11-01), pages 335-346, XP028808712, ISSN: 0163-7258, DOI: 10.1016/J.PHARMTHERA.2013.10.013
- HIROKI ISHIGURO ET AL: "NrCAM-regulating neural systems and addiction-related behaviors : NrCAM and addiction", ADDICTION BIOLOGY, vol. 19, no. 3, 11 May 2014 (2014-05-11), pages 343-353, XP055391874, GB ISSN: 1355-6215, DOI: 10.1111/j.1369-1600.2012.00469.x
- ALEXANDER FASSOLD ET AL: "Soluble neuropilin-2, a nerve repellent receptor, is increased in rheumatoid arthritis synovium and aggravates sympathetic fiber repulsion and arthritis", ARTHRITIS & RHEUMATISM, vol. 60, no. 10, 1 October 2009 (2009-10-01), pages 2892-2901, XP055392363, US ISSN: 0004-3591, DOI: 10.1002/art.24860
- G. P. DEMYANENKO ET AL: "Neural Cell Adhesion Molecule NrCAM Regulates Semaphorin 3F-Induced Dendritic Spine Remodeling", JOURNAL OF NEUROSCIENCE, vol. 34, no. 34, 20 August 2014 (2014-08-20), pages 11274-11287, XP055392365, US ISSN: 0270-6474, DOI: 10.1523/JNEUROSCI.1774-14.2014
- MATTHEW W. PARKER ET AL: "Structural Basis for VEGF-C Binding to Neuropilin-2 and Sequestration by a Soluble Splice Form", STRUCTURE, vol. 23, no. 4, 1 April 2015 (2015-04-01), pages 677-687, XP055392368, AMSTERDAM, NL ISSN: 0969-2126, DOI: 10.1016/j.str.2015.01.018
- K. LEE ET AL: "An Activity-Regulated microRNA, miR-188, Controls Dendritic Plasticity and Synaptic Transmission by Downregulating Neuropilin-2", JOURNAL OF NEUROSCIENCE, vol. 32, no. 16, 18 April 2012 (2012-04-18), pages 5678-5687, XP055391972, US ISSN: 0270-6474, DOI: 10.1523/JNEUROSCI.6471-11.2012

## Description

### BACKGROUND

### 1. Field

One or more embodiments relate to the use of a composition or a kit for use in diagnosing behavioral addiction, and a method of detecting neuropilin-2 or a fragment thereof for diagnosis of behavioral addiction.

### 2. Description of the Related Art

Behavioral addiction relates to addiction involving a certain behavior, process, and/or activity, and examples thereof include gambling addiction, sex addiction, and pathological use of the Internet or game. Behavioral addiction is related to brain circuits, and addiction tolerance and withdrawal symptoms may occur. In addition, impulsivity plays an important role in the early stages of development of behavioral addiction, and obsessive-compulsive behavior is mainly involved in maintaining such behavioral addiction. In modern society, in addition to alcoholism or drug addiction, Internet addiction, game addiction, and obsessive-compulsive behavior has become more prominent.

Biomarkers for psychiatric symptoms, for example, autism, Parkinson's disease, dementia, attention deficit hyperactivity disorder (ADHD), alcoholism, drug addiction, and obsessive-compulsive disorder, have been known in the art (refer to US 2005/0084880 A1 (published on April 21, 2005) and KR 10-1157526 (published on June 27, 2016)). However, regarding diagnosis of behavioral addiction in particular, such as Internet addiction or game addiction, only confirmation by a brain image obtained through magnetic resonance imaging (MRI) or a self-diagnosis method based on a questionnaire survey may be usually used, and no effective biomarkers for such diagnoses have yet been found. Uhl George et al.: "Curious case: Altered dose-response relationships in addiction gene", Pharmacology & Therapeutics, 01 Nov 2013, 141(3):335-346 investigates altered dose-response relationships in addiction genetics.

Therefore, there is a need to develop a biomarker that can be used to diagnose behavioral addiction in an easy and inexpensive way, as well as with high diagnostic accuracy and specificity, instead of using a self-diagnosis method based on subjective judgment or utilizing expensive and inconvenient diagnosis by brain imaging.

### SUMMARY

One or more embodiments include the use of a composition or kit for behavioral addiction diagnosis, the composition or kit comprising an agent for measuring an expression level of neuropilin-2 (NRP-2) protein, or an immunogenic polypeptide part thereof, in a biological sample isolated from a subject.

One or more embodiments include a method of detecting neuropilin-2 (NRP-2) or a part thereof for diagnosis of behavioral addiction, the method comprising: measuring an expression level of NRP-2 protein or an immunogenic polypeptide part thereof in a biological sample isolated from a subject with suspected behavioral addiction; and comparing the measured expression level in the biological sample with that in a normal control group.

The invention is defined in the appended claims.

### BRIEF DESCRIPTION OF THE DRAWINGS

These and/or other aspects will become apparent and more readily appreciated from the following description of the embodiments, taken in conjunction with the accompanying drawings in which:

FIG. 1 is a graph showing results of liquid chromatography in which the high-abundance proteins were removed from a serum sample using immunoaffinity column (x-axis: time (minutes), y-axis: mass absorbance unit (mAU));

FIG. 2A shows images of SDS-PAGE gels obtained before and after depletion of the high-abundance proteins from three serum samples of normal control group , and FIG. 2B shows images of SDS-PAGE gels obtained before and after depletion of the high-abundance proteins from three serum samples of addiction group including Internet or game addicts (wherein left: before depletion of the high-abundance proteins from three individual serum samples, right: after depletion of the high-abundance proteins from three individual serum samples, M: marker size (KDa);

FIG. 3A shows an image obtained by performing a cytokine array on 507 types of proteins, FIG. 3B shows an image obtained by comparing spots of NRP-2 in a control group with those in an addiction group including Internet or game addicts, and FIG. 3C represents a bar graph showing the signal intensities of NRP-2 spots; and

FIG. 4 is a graph showing results obtained by analyzing amounts of NRP-2 from immunoblots of an addiction group including Internet or game addicts (addiction group) or a normal control group (control group).

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

Additional aspects will be set forth in part in the description which follows and, in part, will be apparent from the description, or may be learned by practice of the presented embodiments.

According to the disclosure, a composition for use in diagnosing behavioral addiction includes an agent for measuring an expression level of neuropilin-2 (NRP-2) protein or a fragment thereof.

The term "behavioral addiction" used herein may refer to addiction involving a certain behavior, process, and/or activity. Here, addiction tolerance and withdrawal symptoms may occur, and behavioral addiction may include Internet addiction, game addiction, gambling addiction, sex addiction, and work addiction. Internet addiction may refer to a state of addiction involving a physical, mental, or social hindrance due to pathological or compulsive use of the Internet. Game addiction may refer to a state of addiction involving physical, mental, or social hindrance due to pathological or compulsive immersion in a game. Use of the Internet or games may refer to use of all media including a computer, a mobile phone, a smartphone, and a television.

Neuropilin (NRP) is a protein receptor in neurons. NRP may include an N-terminus CUB domain, coagulation factor 5/8 type, an MAM domain, and a C-terminal neuropilin 4 domain. There are two forms of NRP, NRP-1 and NRP-2. NRP-2 is a protein encoded by a gene, *NRP-2*, in the human body. NRP-2 is a transmembrane protein, and may be bound to proteins SEMA3C and SEMA3F. NRP-2 may interact with vascular endothelial growth factor (VEGF). NRP-2 may also be called NP2, NPN2, PRO2714, or VEGF165R2, and may include an amino acid sequence of Uniprot ID NO. O60462 in humans. NRP-2 may also include an amino acid sequence of Uniprot ID NO. O35375 in mice.

The fragment of the protein used herein refers to a part of NRP-2, and is an immunogenic polypeptide.

The agent used herein may be an antibody or an antigen-binding fragment thereof, which specifically binds to NRP-2 or a fragment thereof. The antibody may be a polyclonal antibody or a monoclonal antibody. The term "antibody" used herein may be used interchangeably with the term "immunoglobulin". The antibody may be a full-length antibody. The antigen-binding fragment may refer to a polypeptide including an antigen-binding site. The antigen-binding fragment may be a single-domain antibody, Fab, Fab', or scFv. The antibody or the antigen-binding fragment thereof may be adhered to a solid support, such as a surface of a metallic chip, plate, or well. The antigen or the antigen-binding fragment thereof may include an anti-NRP-2 antibody or an antigen-binding fragment thereof.

The agent may be nucleic acid including a polynucleotide that is identical to or complementary to a polynucleotide encoding NRP-2 or a fragment thereof. The nucleic acid may be a primer or a probe. The primer or the probe may be labeled with a fluorescent substance, a chemiluminescent substance, or a radioactive isotope, at an end thereof or inside of the primer or the probe.

According to the disclosure, a kit for use in diagnosing behavioral addiction includes an agent for measuring an expression level of neuropilin-2 (NRP-2) protein or an immunogenic polypeptide part thereof.

The kit may further include a sample necessary for diagnosis of behavioral addiction. The kit may include a solid support, and in consideration of immunological detection of the antibody or the antigen-binding fragment thereof, may also include a substrate, a suitable buffer solution, a chromogenic enzyme, a secondary antibody labeled with a fluorescent substance, or a chromogenic substrate. In consideration of detection of nucleic acids, the kit may include a polymerase, a buffering agent, a nucleic acid, a coenzyme, a fluorescent substance, or a combination thereof. The polymerase may be, for example, a Taq polymerase.

According to one or more embodiments, a method of detecting neuropilin-2 (NRP-2) for diagnosis of behavioral addiction includes measuring an expression level of NRP-2 or a fragment thereof in a biological sample isolated from a subject with suspected behavioral addiction; and comparing the measured expression level in the biological sample with that in a normal control group.

The method of detecting NRP-2 includes measuring an expression level of NRP-2 or an immunogenic polypeptide part thereof in a biological sample isolated from a subject with suspected behavioral addiction.

The subject may be a mammal, such as a human being, a cow, a horse, a pig, a dog, a sheep, a goat, or a cat. The subject may suffer from a behavioral addiction or may be suspected of having a behavioral addiction.

The biological sample used herein may refer to a sample obtained from the subject, and examples thereof include blood, plasma, serum, bone marrow fluid, lymph fluid, saliva, tear fluid, mucosal fluid, amniotic fluid, or a combination thereof.

The measuring of the expression level may include incubating the biological sample with an antibody that specifically binds to NRP-2 or an immunogenic polypeptide part thereof.

The measuring of the expression level may be performed by electrophoresis, immunoblotting, enzyme-linked immunosorbent assay (ELISA), protein chip, immunoprecipitation, microarray, northern blotting, polymerase chain reaction (PCR), or a combination thereof. The electrophoresis may be sulfate polyacrylamide gel electrophoresis (SDS-PAGE), isoelectric focusing (IEF), two-dimensional electrophoresis, or a combination thereof. The PCR may be real-time PCR or reverse transcription PCR.

The method of detecting NRP-2 includes comparing the measured expression level in the biological sample with that in a normal control group.

Here, the measured expression level of NRP-2 in the biological sample may be greater than that of NRP-2 derived from a sample isolated from the normal control group. The normal control group may refer to a group not having a behavioral addiction, and is referred to as a negative control group. For example, in the case of a higher expression level of NRP-2 detected in the biological sample than the sample from the normal or negative control group, the subject maybe diagnosed as suffering from or having a high probability of having a behavioral addiction, such as Internet addiction or game addiction. Then, the subject diagnosed with the behavioral addiction, such as Internet addiction or game addiction, may undergo psychosocial therapy or medication. The medication may include, for example, naltrexone, disulfiram, methadone, chlordiazepoxide, acamprosate, bupropion, varenicline, buprenorphine, or a combination thereof.

Hereinafter, the present inventive concept will be described in detail by explaining preferred embodiments of the inventive concept. However, the preferred embodiments should be considered in descriptive sense only and not for purposes of limitation.

### Example 1: Identification of differentially expressed proteins among serum proteins in an addiction group including Internet or game addicts

### 1. Preparation of a sample from addiction group including Internet or game addicts

Serum samples of Internet or game addicts (n=4) were collected from Seoul St. Mary's Hospital of the Catholic University of Korea. In addition, as a negative control group, serum samples of normal subjects (n=4) who were not diagnosed with Internet or game addiction (n=4) were collected.

To prevent protein degradation or modification in serum samples, immediately after obtaining serum, Roche cOmplete™ mini EDTA-free protease inhibitor cocktail and Roche PhosSTOP™ were added as a protease inhibitor and a phosphatase inhibitor, respectively, to the obtained serum. Each inhibitor was prepared according to the manufacturer's protocol, such that 1 tablet of an inhibitor was dissolved in 200 *µℓ* of phosphate buffered saline (PBS) to a concentration of 50 X, and then, was added to each serum sample to obtain a final concentration of 1.5 X. Each serum sample to which the inhibitor was added was quantified, and then, stored at a temperature of 80°C until used in experiments.

### 2. Depletion of high-abundance proteins in serum samples

To remove proteins, such as albumin or immunoglobulin G, that are abundant in serum, serum in the obtained samples was subjected to depletion.

In detail, each of the serum samples obtained in Example 1.1 was prepared in a minimum quantitative amount of 54.8 *µ*g/*µ*ℓ, and then, used for experiments under the same conditions. The prepared serum samples were each diluted as much as 6 times with PBS, and the diluted serum samples were each filtered through a 0.22 *µ*m spin filter. 100 *µ*ℓ of each of the filtrate samples were added to a multiple affinity removal system (MARS) 14 column (Agilent), and then, centrifuged according to the manufacturer's protocol, thereby depleting serum in the samples. Then, a filtrate from which 14 types of abundant proteins (e.g., albumin, immunoglobulin G, alpha-i-antitrypsin, immunoglobulin A, transferrin, haptoglobin, fibrinogen, alpha-2-macroglobulin, alpha-i-acid glycoprotein, immunoglobulin M, apolipoprotein A-I, apolipoprotein A-II, complement protein C3, and transthyretin) were removed was collected from each serum sample.

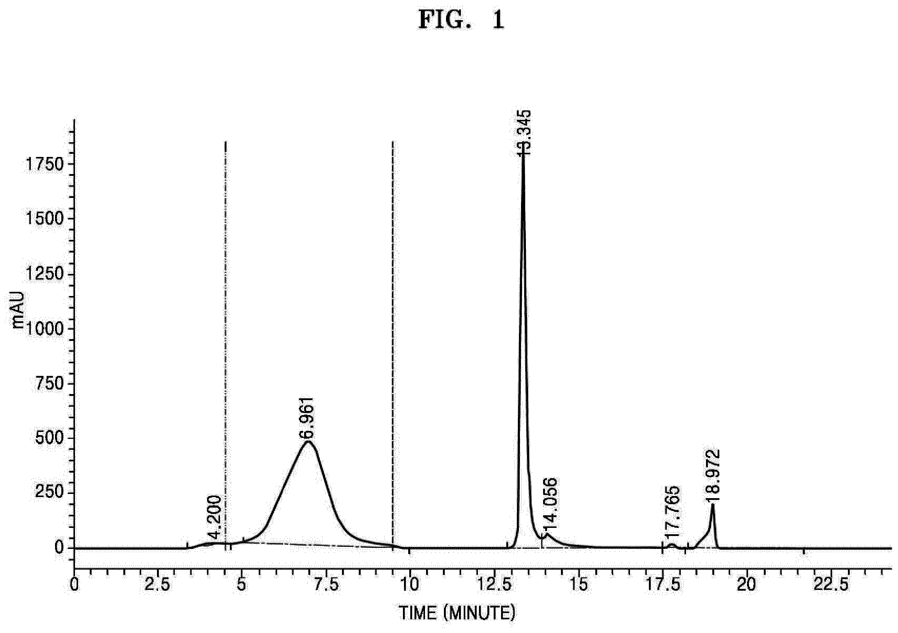

As shown in FIG. 1, each of the serum sample depleted of the top 14 high-abundance proteins was obtained from immunoaffinity depletion (-··-··-: a starting point for collecting the depleted serum, ----: an end point for collecting the depleted serum).

### 3. Concentration and quantification of samples having depleted serum

A centrifugal filter (3K Amicon Ultra, Millipore) was conditioned by adding 400 *µ*ℓ of water (HPLC grade, J.T Baker) twice and 400 *µ*ℓ of 10 mM HEPES buffer solution (pH 8.0) twice.

The samples having depleted serum obtained in Example 1.2 were each diluted as much as 5 times with a 10 mM HEPES buffer solution, and then, were each centrifuged in the prepared centrifugal filter at 14,000xg for 10 minutes at a temperature of 10°C, thereby concentrating each of the samples. Afterwards, the centrifugal filter was washed with a 10 mM HEPES buffer solution 5 times, and then, the washed centrifugal filter containing concentrated contents was inverted and contents thereof were added into a new tube.

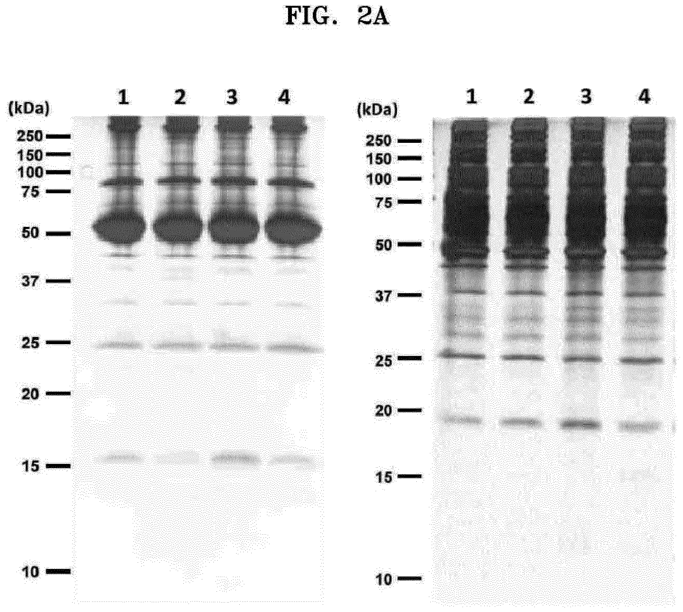

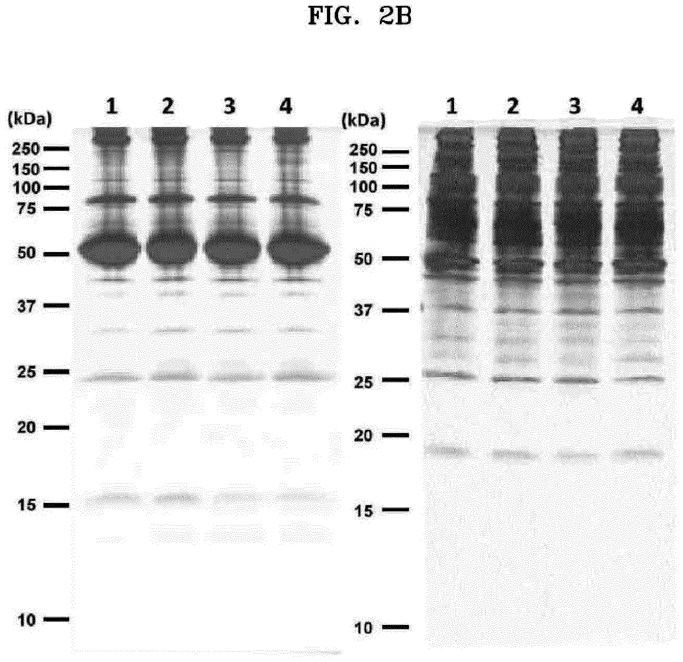

The concentrated samples were each quantified using a BCA assay, and 2 µg of each sample was separated by SDS-PAGE electrophoresis. Then, the electrophoresed gel was stained by a silver-stain method. FIGS. 2A and 2B show images of the stained gel (FIG. 2A: images of the SDS-PAGE electrophoresis gel obtained before and after subjecting serum samples in a normal control group to depletion, FIG. 2B: images of the SDS-PAGE electrophoresis gel obtained before and after subjecting serum samples in an addiction group including Internet or game addicts to depletion).

### 4. Comparative analysis of protein expression from normal and addiction samples using antibody arrays

4 serum samples of the normal control group and 4 serum samples of the addiction group including Internet or game addicts were pooled to a concentration of 500 *µ*g/ 1.5 ml of total volumes.

3.6 *µ*ℓ of biotinylation agent (available from RayBiotech, Inc.) were added to each of the pooled serum samples, incubated for 30 minutes at room temperature, and then, 5 *µ*ℓ of a reaction termination solution was added thereto to terminate the reaction. The reaction product was filtered through a size exclusion column (available from RayBiotech, Inc.), thereby removing the remaining biotin in each the serum samples.

The human antibody 507 arrays (membrane type, available from RayBiotech, Inc.) were blocked for 1 hour at room temperature by a blocking buffer solution. PBS was added to 1.5 mℓ of each of the biotin-labeled samples to dilute to a total volume of 14 mℓ. Each of the diluted samples was then added to the array, and each array was incubated overnight at a temperature of about 4°C.

Afterwards, a washing buffer solution included in an array kit was used to wash the array, a streptavidin-HRP conjugate solution (available from RayBiotech, Inc.) was added to the array, and then, the array was incubated for 2 hours at room temperature. The antibody array was washed again, and signals from the array were detected using chemiluminescence.

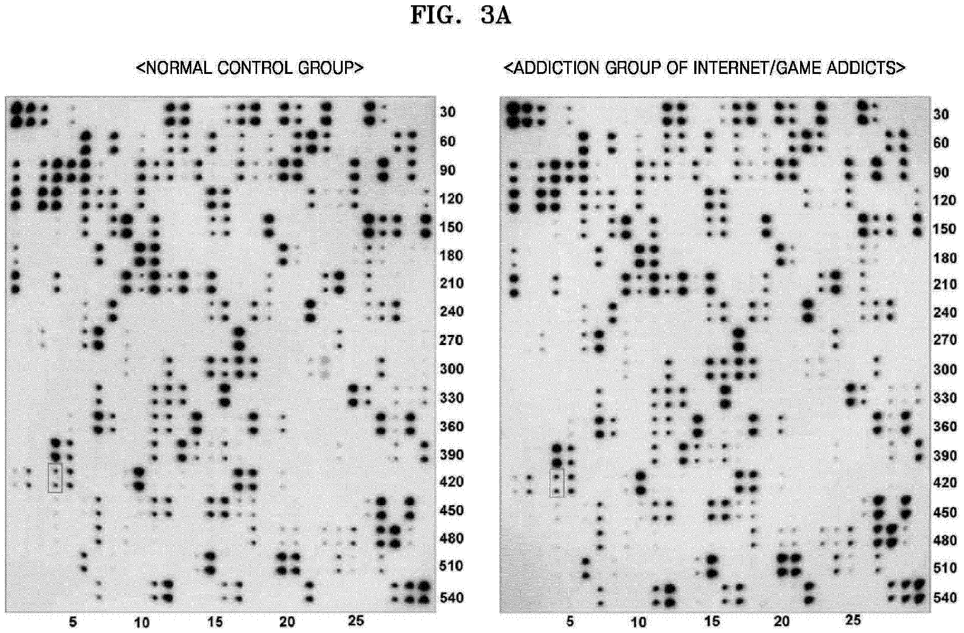

The results of the antibody array containing 507 kinds of antibodies are shown in FIG. 3A. As shown in FIG. 3A, several proteins showing differential expression were observed in the normal control group and the addiction group including Internet or game addicts.

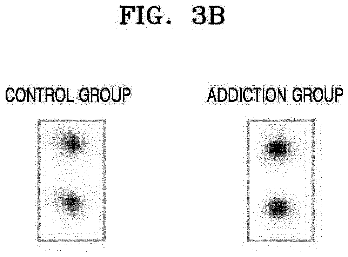

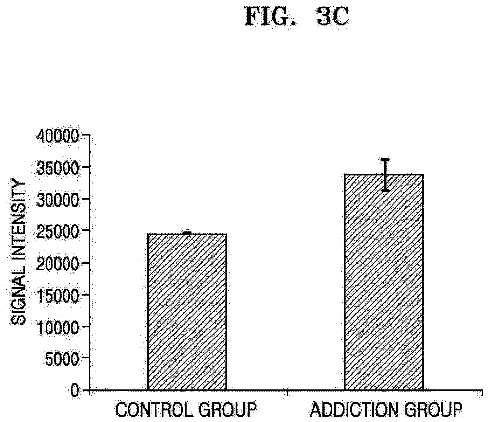

Among the proteins that were expressed differentially in the addiction group including Internet or game addicts, NRP-2 was chosen to be a biomarker. An image of the cytokine array for NRP-2 is shown in FIG. 3B. The signal was quantified using TotalLab 1D analysis software (Nonlinear Dynamics) as seen in FIG. 3C.

### 5. Verification of differentially expressed proteins using immunoblotting

The expression pattern of NRP-2, which was chosen to be a biomarker candidate in Example 1.4, was verified. To do this, Seoul St. Mary's Hospital of the Catholic University of Korea provided 34 samples as a control group and 27 samples as an addiction group including Internet or game addicts to carry out experiments.

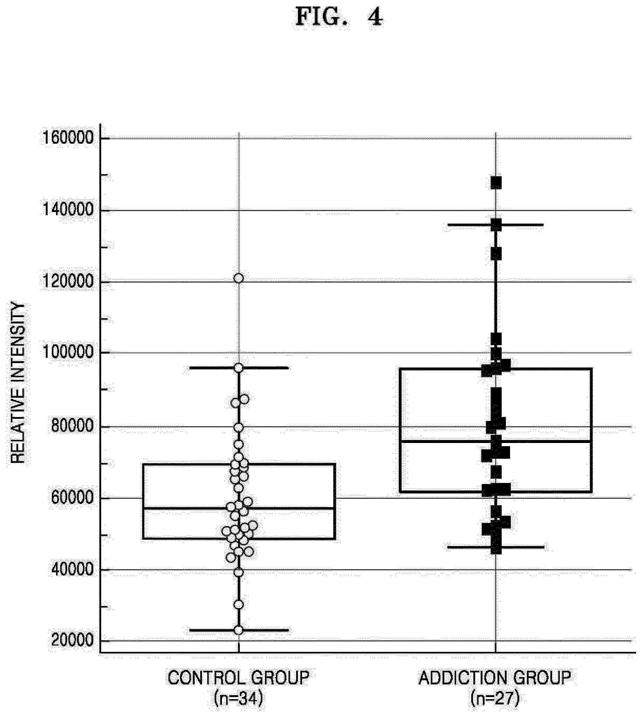

In both the control group and the addiction group, 35 *µ*g of a protein sample were subjected to electrophoresis, followed by immunoblotting using anti-NRP-2 antibodies (available from Abeam) and secondary antibodies (available from Santacruz biotechnology). Images obtained by immunoblotting were analyzed, and the results obtained by a Mann-Whitney U test method are shown in FIG. 4 (p=0.001).

As shown in FIG. 4, it was confirmed that the expression of NRP-2 increased in the addiction group including Internet or game addicts as compared with that in the normal control group, and that is, NRP-2 showed excellent accuracy as a biomarker for Internet or game addiction.

As described above, the use of a composition or a kit for diagnosing behavioral addiction and a method of detecting neuropilin-2 (NRP-2) for diagnosis of behavioral addiction may be used to diagnose behavioral addiction, for example, Internet addiction or game addiction, in an easy, and highly accurate, with high specificity and high sensitivity.

It should be understood that embodiments described herein should be considered in a descriptive sense only and not for purposes of limitation. Descriptions of features or aspects within each embodiment should typically be considered as available for other similar features or aspects in other embodiments.

## Claims

1. Use of a composition or kit for behavioral addiction diagnosis, the composition or kit comprising an agent for measuring an expression level of neuropilin-2 (NRP-2) protein, or an immunogenic polypeptide part thereof, in a biological sample isolated from a subject.

2. The use of claim 1, wherein the behavioral addiction is selected from the group consisting of Internet addiction, game addiction, gambling addiction, sex addiction, shopping addiction, and work addiction.

3. The use of claim 1 or claim 2, wherein the agent is an antibody or an antigen-binding fragment thereof, which specifically binds to NRP-2 protein or an immunogenic polypeptide part thereof; or
a nucleic acid including a polynucleotide identical to or complementary to a polynucleotide encoding NRP-2 protein or an immunogenic polypeptide part thereof.

4. The use of claim 3, wherein the antibody is a polyclonal antibody or a monoclonal antibody.

5. The use of claim 3, wherein the nucleic acid is a primer or a probe.

6. A method of detecting NRP-2 protein or a fragment thereof for diagnosis of behavioral addiction, the method comprising:
measuring an expression level of NRP-2 protein or an immunogenic polypeptide part thereof in a biological sample isolated from a subject with suspected behavioral addiction; and
comparing the measured expression level in the biological sample with that in a normal control group.

7. The method of claim 6, wherein the biological sample includes blood, plasma, serum, bone marrow fluid, lymph fluid, saliva, tear fluid, mucosal fluid, amniotic fluid, or a combination thereof.

8. The method of claim 6, wherein the measuring of the expression level comprises incubating the biological sample with an antibody that specifically binds to NRP-2 protein or an immunogenic polypeptide part thereof.

9. The method of claim 6, wherein the measuring of the expression level is performed by electrophoresis, immunoblotting, enzyme-linked immunosorbent assay (ELISA), protein chip, immuno-precipitation, microarray, Northern blotting, polymerase chain reaction (PCR), or a combination thereof.

## Patentansprüche

1. Verwendung einer Zusammensetzung oder eines Kits für Verhaltenssucht-Diagnose, die Zusammensetzung oder das Kit umfassend ein Mittel zum Messen eines Expressionsniveaus von Protein Neuropilin-2 (NRP-2) oder einen immunogenen Polypeptidabschnitt davon in einer biologischen Probe, die von einem Subjekt isoliert ist.

2. Verwendung nach Anspruch 1, wobei die Verhaltenssucht ausgewählt ist aus der Gruppe bestehend aus Internetsucht, Spielsucht, Glücksspielsucht, Sexsucht, Kaufsucht und Arbeitssucht.

3. Verwendung nach Anspruch 1 oder Anspruch 2, wobei das Mittel ein Antikörper oder ein antigenbindendes Fragment davon ist, das spezifisch an das Protein NRP-2 oder einen immunogenen Polypeptidabschnitt davon bindet; oder
eine Nukleinsäure, die ein Polynukleotid beinhaltet, das identisch wie ein Polynukleotid oder komplementär dazu ist, das für ein Protein NRP-2 oder einen immunogenen Polypeptidabschnitt davon kodiert.

4. Verwendung nach Anspruch 3, wobei der Antikörper ein polyklonaler Antikörper oder ein monoklonaler Antikörper ist.

5. Verwendung nach Anspruch 3, wobei die Nukleinsäure ein Primer oder eine Sonde ist.

6. Verfahren zum Nachweisen von Protein NRP-2 oder einem Fragment davon zur Diagnose von Verhaltenssucht, das Verfahren umfassend:
Messen eines Expressionsniveaus von Protein NRP-2 oder eines immunogenen Polypeptidabschnitts davon in einer biologischen Probe, die von einem Subjekt mit vermuteter Verhaltenssucht isoliert ist; und
Vergleichen des gemessenen Expressionsniveaus in der biologischen Probe mit dem in einer normalen Kontrollgruppe.

7. Verfahren nach Anspruch 6, wobei die biologische Probe Blut, Plasma, Serum, Knochenmarkflüssigkeit, Lymphflüssigkeit, Speichel, Tränenflüssigkeit, Schleimhautflüssigkeit, Amnionflüssigkeit oder eine Kombination davon beinhaltet.

8. Verfahren nach Anspruch 6, wobei das Messen des Expressionsniveaus ein Inkubieren der biologischen Probe mit einem Antikörper umfasst, der spezifisch an Protein NRP-2 oder einen immunogenen Polypeptidabschnitt davon bindet.

9. Verfahren nach Anspruch 6, wobei das Messen des Expressionsniveaus durch Elektrophorese, Immunblotting, Enzym-Immunassay (ELISA), Proteinchip, Immunpräzipitation, Microarray, Northern Blotting, Polymerasekettenreaktion (PCR) oder eine Kombination davon ausgeführt wird.

## Revendications

1. Utilisation d'une composition ou d'une trousse permettant le diagnostic de l'accoutumance comportementale, la composition ou la trousse comprenant un agent permettant de mesurer le niveau d'expression de la protéine neuropiline-2 (NRP-2), ou d'une partie polypeptidique immunogène de celle-ci, dans un échantillon biologique isolé à partir d'un sujet.

2. Utilisation selon la revendication 1, ladite accoutumance comportementale étant choisie dans le groupe constitué par la dépendance à Internet, la dépendance aux jeux, la dépendance aux jeux d'argent, la dépendance au sexe, la dépendance au shopping et la dépendance au travail.

3. Utilisation selon la revendication 1 ou la revendication 2, ledit agent étant un anticorps ou un fragment de liaison à l'antigène de celui-ci, qui se lie spécifiquement à la protéine NRP-2 ou à une partie polypeptidique immunogène de celle-ci ; ou
un acide nucléique comprenant un polynucléotide identique ou complémentaire à un polynucléotide codant la protéine NRP-2 ou une partie polypeptidique immunogène de celle-ci.

4. Utilisation selon la revendication 3, ledit anticorps étant un anticorps polyclonal ou un anticorps monoclonal.

5. Utilisation selon la revendication 3, ledit acide nucléique étant une amorce ou une sonde.

6. Méthode de détection de la protéine NRP-2 ou d'un fragment de celle-ci pour le diagnostic de l'accoutumance comportementale, la méthode comprenant :
la mesure du niveau d'expression de la protéine NRP-2 ou d'une partie polypeptidique immunogène de celle-ci dans un échantillon biologique isolé à partir d'un sujet soupçonné d'accoutumance comportementale ; et
la comparaison du niveau d'expression mesuré dans l'échantillon biologique à celui d'un groupe témoin normal.

7. Méthode selon la revendication 6, ledit échantillon biologique comprenant du sang, du plasma, du sérum, du fluide de moelle osseuse, du liquide lymphatique, de la salive, du liquide lacrymal, du fluide des muqueuses, du liquide amniotique ou une combinaison de ceux-ci.

8. Méthode selon la revendication 6, ladite mesure du niveau d'expression comprenant l'incubation de l'échantillon biologique avec un anticorps qui se lie spécifiquement à la protéine NRP-2 ou à une partie polypeptidique immunogène de celle-ci.

9. Méthode selon la revendication 6, ladite mesure du niveau d'expression étnt effectuée par électrophorèse, immunotransfert, dosage immunoenzymatique (ELISA), puce protéique, immuno-précipitation, puce à ADN, transfert de Northern, amplification en chaîne par polymérase (ACP) ou une combinaison de ceux-ci.
